(19) Europäisches Patentamt
European Patent Office
Office européen des brevets

(11) Numéro de publication : **0 070 787**
**B1**

(12) **FASCICULE DE BREVET EUROPÉEN**

(45) Date de publication du fascicule du brevet : **26.06.85**

(51) Int. Cl.⁴ : **C 07 C 51/56**, C 07 C 53/12

(21) Numéro de dépôt : **82420094.3**

(22) Date de dépôt : **09.07.82**

(54) **Procédé de carbonylation de l'acétate de méthyle.**

(30) Priorité : **17.07.81 FR 8114123**

(43) Date de publication de la demande : **26.01.83 Bulletin 83/04**

(45) Mention de la délivrance du brevet : **26.06.85 Bulletin 85/26**

(84) Etats contractants désignés : **AT BE CH DE FR GB IT LI LU NL SE**

(56) Documents cités :
DE-A- 933 148
DE-A- 1 006 847
US-A- 2 593 440
US-A- 2 727 902

(73) Titulaire : **RHONE-POULENC CHIMIE DE BASE**
**25, quai Paul Doumer**
**F-92408 Courbevoie (FR)**

(72) Inventeur : **Doussain, Claude**
**2, rue Louis Girardet**
**F-69190 Saint-Fons (FR)**
Inventeur : **Gauthier-Lafaye, Jean**
**21, rue Duguesclin**
**F-69006 Lyon (FR)**
Inventeur : **Perron, Robert**
**La Pécollère**
**F-69390 Charly (FR)**

(74) Mandataire : **Varnière-Grange, Monique et al**
**RHONE-POULENC RECHERCHES Service Brevets Chimie et Polymères Centre de Recherches de Saint-Fons 85, avenue des Frères Perret B.P. 62**
**F-69192 St-Fons Cedex (FR)**

**EP 0 070 787 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de carbonylation de l'acétate de méthyle.

La présente invention a plus particulièrement pour objet un perfectionnement au procédé de carbonylation de l'acétate de méthyle, mettant en œuvre un catalyseur à base de cobalt.

Le brevet américain n° 2 730 546 décrit la carbonylation de l'acétate de méthyle en anhydride acétique en présence d'un catalyseur choisi parmi les complexes du cobalt de formule générale :

$$[R_4A]_2CoX_4$$

dans laquelle X représente un atome de brome ou d'iode, A un atome d'azote ou de phosphore et R représente un radical alkyle inférieur, par exemple.

Ces complexes peuvent être formés in-situ en chargeant d'une part un halogénure du cobalt ($CoX'_2$) et d'autre part un halogénure d'ammonium (ou de phosphonium) quaternaire ($R_4AX$). La formation des complexes en question peut alors être représentée par la réaction suivante :

$$2(R_4AX) + CoX'_2 \longrightarrow [R_4A]_2CoX'_2X_2$$

Toutefois, l'efficacité de ces catalyseurs à base de cobalt paraît relativement faible. Ce type de procédé, dont l'intérêt de principe n'est pas contesté, n'a pu déboucher sur une réalisation industrielle.

Le brevet français n° 2 242 362 (correspondant aux demandes américaines n° 394 220 et n° 467 977, respectivement du 4 septembre 1973 et du 8 mai 1974) décrit un procédé de préparation de l'anhydride acétique en deux stades, dans lequel, lors d'une première étape, le bromure, ou de préférence l'iodure, de méthyle est carbonylé pour obtenir l'halogénure d'acétyle correspondant, cet halogénure d'acétyle étant à son tour mis à réagir sur de l'acétate de méthyle pour conduire, dans une deuxième étape, à l'anhydride acétique, ce qui correspond au schéma réactionnel suivant, dans le cas où l'iodure de méthyle est la matière de départ :

— Etape 1 :

$$CH_3I + CO \longrightarrow CH_3COI$$

— Etape 2 :

$$CH_3COI + CH_3COOCH_3 \longrightarrow (CH_3CO)_2O + CH_3I$$

Comme ce schéma le fait apparaître clairement, l'iodure de méthyle, matière de départ de l'étape 1, est « régénéré » dans l'étape 2. L'étape 1 est avantageusement conduite en présence de catalyseur à base de rhodium ; l'étale 2 serait favorisée par la présence de lithium et/ou de chrome. Les deux étapes peuvent être réalisées dans une même zone réactionnelle qui renfermera alors de l'iodure de méthyle, de l'acétate de méthyle, du rhodium et le cas échéant du lithium et/ou du chrome, voire de l'iodure d'acétyle, zone dans laquelle on introduira également du monoxyde de carbone.

Le brevet américain n° 4 115 444 propose un perfectionnement de la technique décrite dans le brevet français précité, perfectionnement qui consiste à ajouter au milieu réactionnel un composé organique du phosphore ou de l'azote, dans lequel le phosphore ou l'azote est trivalent. Ce brevet confirme l'intérêt potentiel des systèmes catalytiques à base de rhodium, voire de palladium ou d'iridium, et de chrome dans cette réaction.

Le brevet français n° 2 303 788 (correspondant aux demandes américaines n° 556 750 et n° 654 661 respectivement du 10 mars 1975 et du 5 février 1976) montre que la présence d'une quantité importante d'hydrogène dans le milieu réactionnel défini ci-avant influe de manière notable sur l'orientation de la réaction. En effet on obtient dans ces conditions un mélange renfermant une proportion prépondérante d'acide acétique, des quantités variables de diacétate d'éthylidène, d'anhydride acétique et d'acétaldéhyde.

L'intérêt principal de ces procédés mettant en œuvre des catalyseurs à base de rhodium, voire de palladium ou d'iridium, les systèmes basés sur le couple (rhodium-chrome) paraissant être les plus actifs, réside essentiellement dans la possibilité qu'ils offrent, d'accéder à l'anhydride acétique au départ d'acétate de méthyle en utilisant des pressions partielles de monoxyde de carbone plus basses que celles requises dans les procédés antérieurs.

Néanmoins, les tentatives de développement de ce type de procédés, même sur une simple échelle pré-industrielle, se heurtent à des difficultés non-négligeables.

Une première série de difficultés réside dans le fait que les catalyseurs à base de rhodium ou de palladium, voir d'iridium, métaux extrêmement rares et coûteux, se désactivent notamment lors des traitements nécessaires à la récupération du (ou des) produit(s) de la réaction. En raison du coût de ces catalyseurs il est indispensable de les régénérer. Pair ailleurs les pertes en rhodium qui paraissent

inévitables dans les divers points d'une unité industrielle grèvent lourdement l'économie d'un tel procédé.

Une seconde série de difficultés trouve sa source dans la présence, nécessaire au bon déroulement de la réaction et à la stabilisation du rhodium, de grandes quantités d'iodure de méthyle (ou d'acétyle) qui implique des risques importants de corrosion dans les divers points d'une installation industrielle. Par ailleurs l'iodure de méthyle et/ou certains de ses dérivés formés dans le milieu réactionnel sont responsables d'une contamination intolérable du (ou des) produit(s) réactionnel(s) qui nécessite la mise en œuvre d'étapes supplémentaires pour éliminer les iodures dont la présence s'avère indésirable dans les produits de réaction. Ces dérivés iodés présents en grandes quantités non seulement dans les produits mais aussi dans divers effluents issus de la zone réactionnelle doivent, pour des raisons économiques évidentes, être récupérés ce qui implique des stades de traitement additionnels.

Les divers problèmes, délicats à résoudre, associés à ce type de procédés apparaîtront plus clairement à la lecture des brevets français n° 2 438 023 et n° 2 438 024 (correspondant respectivement aux demandes américaines n° 949 344 et n° 949 345 déposées le 6 octobre 1978) et du brevet américain n° 4 241 219.

Par ailleurs, il est bien connu que l'acétate de méthyle peut être obtenu par réaction de l'acide acétique et du méthanol, l'acide acétique pouvant être produit par carbonylation du méthanol et le méthanol pouvant être à son tour fabriqué par hydrogénation du monoxyde de carbone. Les réactions en cause sont susceptibles d'être représentées comme suit :

$$CO + 2H_2 \longrightarrow CH_3OH \quad (a)$$
$$CH_3OH + CO \longrightarrow CH_3COOH \quad (b)$$
$$CH_3COOH + CH_3OH \longrightarrow CH_3COOCH_3 + H_2O \quad (c)$$

La carbonylation de l'acétate de méthyle en milieu sensiblement anhydre permet d'obtenir l'anhydride acétique selon la réaction suivante :

$$CH_3COOCH_3 + CO \longrightarrow CH_3CO{-}O{-}OCH_3 \quad (1)$$

Ainsi, l'intérêt d'un procédé de carbonylation de l'acétate de méthyle en anhydride acétique (1) apparaît clairement lorsqu'on considère également les réactions (a) à (c) ci-avant, puisque globalement cet ensemble revient à un procédé par lequel l'anhydride acétique est produit au départ de monoxyde de carbone et d'hydrogène.

D'autre part, le cobalt étant un métal courant, on conçoit aisément l'intérêt potentiel de son utilisation dans un procédé de carbonylation de l'acétate de méthyle. Il est donc étonnant de constater que toutes les solutions antérieures proposées récemment reposent sur la préconisation de systèmes catalytiques à base de rhodium.

Il a maintenant été trouvé de manière tout à fait surprenante qu'il est possible de carbonyler efficacement l'acétate de méthyle, en faisant appel à un système catalytique à base de cobalt.

La présente invention a donc pour objet un procédé de préparation de l'anhydride acétique par réaction de l'acétate de méthyle et du monoxyde de carbone en phase liquide, en milieu anhydre en présence :

a) d'une source de cobalt et d'un promoteur halogéné, caractérisé en ce que la réaction est conduite à une température comprise entre 80 et 240 °C sous une pression totale comprise entre 20 et 300 bars en présence également :

b) d'une source de fer au degré d'oxydation nul,

c) d'un iodure ionique de formule :

$$A^+I^-$$

dans laquelle $A^+$ est un cation choisi dans le groupe constitué par les cations onium quaternaire dérivant des éléments du groupe de l'azote et les cations des métaux alcalins,

d) le cas échéant, d'un carboxylate de formule :

$$A'^{n+}(OCOR)_n^-$$

dans laquelle n est égal à 1 ou 2 et $A'^{n+}$ a la signification donnée ci-avant pour $A^+$, $A'^{n+}$ et $A^+$ pouvant par ailleurs être identiques ou différents, $A'^{n+}$ pouvant, en outre, représenter un cation alcalino-terreux, et R est un radical alkyle, aralkyle ou aryle ayant au maximum 8 atomes de carbone, la quantité totale de composés halogénés présente dans le milieu réactionnel (exprimée en atome-gramme d'halogène et désignée par $X_T$ dans ce qui suit) étant telle que le rapport atomique $X_T/(A^+ + n \cdot A'^{n+})$ soit inférieur ou égal à 1.

Le présent procédé de carbonylation de l'acétate de méthyle est remarquable en divers points. En effet il a été trouvé de manière tout à fait inattendue que l'adjonction d'une source de fer au degré d'oxydation nul au milieu réactionnel renfermant des ingrédients du type (a), du type (c) et, le cas

échéant, du type (d) mentionnés ci-avant, permet d'obtenir notamment de l'anhydride acétique, avec une productivité horaire particulièrement élevée, alors que dans les mêmes conditions réactionnelles, le cobalt, seul, ne possède qu'une médiocre activité carbonylante dans la réaction envisagée et que le fer seul, dans ces mêmes conditions, ne développe pratiquement aucune activité carbonylante.

La demanderesse, sans pour autant être liée par une quelconque explication (ou mécanisme réactionnel), est conduite par ces faits surprenants à penser que l'activité carbonylante doit être attribuée, dans le présent procédé, à la source de cobalt qui, dans les conditions réactionnelles, se transformerait en une espèce catlytiquement active, dont la nature n'est pas totalement élucidée.

Le second constituant métallique du système (source de fer au degré d'oxydation nul), qui ne possède en lui-même pratiquement aucune activité carbonylante, ne servirait apparamment qu'à augmenter l'activité et/ou la concentration en espèce active du cobalt ou sa durée de vie, le cas échéant. La raison de cette influence remarquable est inconnue mais pourrait cependant résider dans un changement du degré d'oxydation et/ou de la disposition, voire de la nature, des coordinats de l'atome central de cobalt. En d'autres termes, la source de fer au degré d'oxydation nul aurait pour fonction de faciliter l'apparition dans le milieu réactionnel de la forme active du cobalt.

Par ailleurs il a été trouvé de manière tout à fait surprenante que, contrairement à l'enseignement de l'art antérieur, l'efficacité du présent procédé n'est pas liée à la présence de grandes quantités d'iodure de méthyle. Au contraire, lorsqu'on ajoute de l'iodure de méthyle lors de la mise en œuvre du présent procédé, on observe une baisse notable de son efficacité.

La raison précise de l'influence négative de l'iodure de méthyle, phénomène en contradiction totale avec l'enseignement de l'art antérieur, est inconnue. La Demanderesse, sans pour autant être liée par une quelconque explication, suppose que l'iodure de méthyle, lorsqu'il est présent en quantités notables, rompt les équilibres complexes qui seraient notamment à l'origine de la très grande réactivité du cobalt dans les conditions décrites.

Par contre l'activité carbonylante du système précédemment défini n'est pas liée à la nature précise du ou des composés du cobalt chargés initialement. N'importe quelle source de cobalt susceptive de réagir avec l'oxyde de carbone dans le milieu de réaction pour donner des complexes carbonyles du cobalt peut être utilisée dans le cadre du présent procédé. Les sources typiques du cobalt sont par exemple le cobalt métallique finement divisé, des sels inorganiques de cobalt (nitrate, carbonate, halogénures ...) ou des sels organiques en particulier des carboxylates. Peuvent également être employés les cobalt-carbonyles ou hydrocarbonyles. Parmi les dérivés du cobalt convenant à la mise en œuvre du procédé selon l'invention, on peut citer le formiate, l'acétate et plus particulièrement le dicobaltoctacarbonyle.

La quantité précise de cobalt engagée à la réaction n'est pas fondamentale. En général, on opère avec une quantité de cobalt telle que la concentration dans le milieu réactionnel exprimée en milliatomes-grammes par litre (mAt-g/l) soit comprise entre 0,1 et 500 et de préférence entre 0,5 et 100 mAt-g/l.

Un avantage du présent procédé réside dans le fait qu'il est possible d'obtenir de bons résultats avec une faible concentration en cobalt.

Pour mettre en œuvre le présent procédé il est également fait appel à une source de fer au degré d'oxydation nul. Cette source de fer au degré d'oxydation nul peut être du fer métallique, du fer pentacarbonyle ou un alliage renfermant divers métaux, dans lequel le fer représente au moins 20 % (en poids). A titre d'exemple d'alliages convenant à la mise en œuvre du présent procédé on peut citer les aciers inoxydables, notamment l'acier Z8 CNDT 17-12 (norme Afnor), et des alliages fer-chrome.

Le fer métallique ou ses alliages peuvent être utilisés sous toute forme commode. Ainsi on peut recourir à des poudres, à de la limaille, à des fils éventuellement tressés, à des blocs solides, à des cylindres ou à des tampons. Par ailleurs, si les parois de l'autoclave (ou du réacteur), dans l'hypothèse où celui-ci serait conçu en acier inoxydable par exemple, peuvent apporter une contribution non négligeable au déroulement du présent procédé, cette contribution étant toutefois insuffisante, il conviendra de faire également appel, dans cette hypothèse, à une autre source de fer au degré d'oxydation nul.

La quantité de fer à mettre en œuvre est en général telle que le rapport atomique Fe/Co soit supérieur ou égal à 1 et, de préférence, supérieur ou égal à 5. Pour une bonne mise en œuvre du présent procédé le rapport atomique Fe/Co sera supérieur ou égal à 10. Il n'est pas utile que ce rapport dépasse 1 000.

Le procédé selon la présente invention nécessite également la présence d'un iodure de formule :

$$A^+I^-$$

dans laquelle $A^+$ est un cation choisi dans le groupe constitué par les cations onium quaternaire dérivant des éléments du groupe de l'azote et les cations des métaux alcalins.

Par cations onium quaternaire dérivés des éléments du groupe de l'azote on entend des cations formés à partir d'azote, de phosphore, d'arsenic ou d'antimoine et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical unique divalent.

Parmi ces composés la Demanderesse préconise l'emploi d'iodures de phosphonium (ou d'ammonium) quaternaire. Les cations de ces iodures peuvent être représentés de manière commode par les

formules (I) à (III) ci-après :

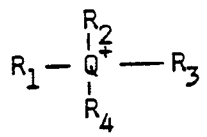

(I)

dans laquelle Q représente un atome d'azote ou de phosphore, $R_1$, $R_2$, $R_3$ et $R_4$, pouvant être identiques ou différents représentent des radicaux organiques dont la valence libre est portée par un atome de carbone, deux quelconques de ces divers radicaux pouvant éventuellement former ensemble un radical unique divalent.

Plus spécifiquement $R_1$, $R_2$, $R_3$ et $R_4$ peuvent représenter des radicaux alkyles linaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux alkyles ayant de 1 à 4 atomes de carbone ; deux des radicaux $R_1$ à $R_4$ pouvant éventuellement former ensemble un radical unique divalent — alkylène ou alcénylène — comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

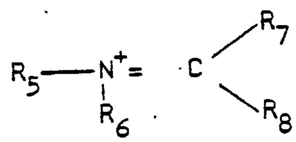

(II)

dans laquelle $R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple ; $R_6$ et $R_7$ ou $R_8$ peuvent former ensemble un radical unique divalent — alkylène ou alcénylène — comportant 4 atomes de carbone et le cas échéant 1 ou 2 doubles liaisons éthyléniques, l'atome d'azote étant alors inclus dans un hétérocycle, pour constituer, par exemple, un cation pyridinium.

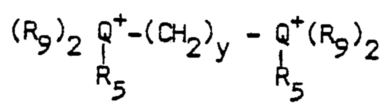

(III)

dans laquelle $R_5$ et $Q^+$ ont la signification donnée précédemment, $R_9$ pouvant être identique à $R_5$ représente un radical alkyle ayant de 1 à 4 atomes de carbone ou un radical phényle, et y est un entier compris entre 1 et 10 inclus et de préférence entre 1 et 6 inclus. A titre d'exemple d'iodures d'ammonium quaternaire convenant à la mise en œuvre du présent procédé on peut citer les iodures

de tétraméthylammonium,
de triéthylméthylammonium,
de tributylméthylammonium,
de triméthyl(n-propyl)ammonium,
de tétraéthylammonium,
de tétrabutylammonium,
de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylènammonium,
de N,N-diéthyl-triméthylènammonium,
de N,N-diméthyl-tétraméthylènammonium,
de N,N-diéthyl-tétraméthylènammonium,
de N-méthylpyridinium,
de N-éthylpyridinium,
de N-méthylpicolinium.

A titre d'exemple d'iodures de phosphonium quaternaire convenant également à la mise en œuvre du présent procédé, on peut citer les iodures

de tétraméthylphosphonium,
d'éthyltriméthylphosphonium,
de triméthylpentylphosphonium,
d'octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri(isopropyl)phosphonium,
de méthyl-tri(n-propyl)phosphonium,
de méthyl-tri(n-butyl)phosphonium,
de méthyl-tri(méthyl-2 propyl)phosphonium,
de méthyltricyclohexylphosphonium,
de méthyltriphénylphosphonium,
de méthyltribenzylphosphonium,
de méthyl-tri(méthyl-4 phényl)phosphonium,
de méthyltrixylylphosphonium,
de diéthylméthylphénylphosphonium,
de dibenzylméthylphénylphosphonium,
d'éthyltriphénylphosphonium,
de tétraéthylphosphonium,
d'éthyl-tri(n-propyl)phosphonium,
de triéthylpentylphosphonium,
d'éthyltriphénylphosphonium,
de n-butyl-tri(n-propyl)phosphonium,
de butyltriphénylphosphonium,
de benzyltriphénylphosphonium,
de (β-phényléthyl)diméthylphénylphosphonium,
de tétraphénylphosphonium,
de triphényl(méthyl-4 phényl)phosphonium.

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les iodures d'ammonium ou de phosphonium quaternaire représentés soit par la formule (I) dans laquelle l'un quelconque des radicaux $R_1$ à $R_4$ est choisi parmi les radicaux alkyles linéaires ayant de 1 à 4 atomes de carbone, soit par les formules (II) ou (III) dans laquelle $R_5$ (ou $R_6$) est également un radical alkyle ayant de 1 à 4 atomes de carbone, conviennent particulièrement bien.

En outre, parmi les iodures d'ammonium on préfère ceux dont les cations correspondent à la formule (I) dans laquelle tous les radicaux $R_1$ à $R_4$ sont choisis parmi les radicaux alkyles linéaires ayant de 1 à 4 atomes de carbone et dont au moins trois d'entre eux sont identiques.

De même, parmi les iodures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres radicaux étant identiques et choisis parmi les radicaux phényle, tolyle ou xylyle.

Les iodures des métaux alcalins conviennent également à la mise en œuvre de la présente invention. Toutefois dans le cas où de tels iodures sont mis en œuvre il convient d'introduire également dans le milieu réactionnel un solvant choisi parmi la tétraméthylènesulfone, la tétraméthylurée, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone. Ce type de solvant est utilisé à raison de 10 à 50 % (en volume) du milieu réactionnel, bien que des proportions supérieures ou inférieures puissent être mises en œuvre.

La quantité d'iodure ionique à mettre en œuvre dans le cadre du présent procédé est en général telle que le rapport molaire $I^-/Co$ soit supérieur ou égal à 5 et de préférence, supérieur ou égal à 10. Il n'est pas utile que ce rapport dépasse la valeur de 200. On fixera avantageusement le rapport molaire $I^-/Co$ à une valeur comprise entre 15 et 100.

Comme indiqué en tête du présent mémoire, la présente invention peut être mise en œuvre également en présence d'un carboxylate de formule :

$$A'^{n+}(OCOR)_n^-$$

dans laquelle n est égal à 1 ou 2 et $A'^{n+}$ a la signification donnée ci-avant pour $A^+$, $A'^{n+}$ et $A^+$ pouvant par ailleurs être identiques ou différents, $A'^{n+}$ pouvant, en outre, représenter un cation alcalino-terreux, et R est un radical alkyle, aralkyle ou aryle ayant au maximum 8 atomes de carbone.

Ce mode d'exécution constitue une variante avantageuse du présent procédé lorsqu'on fait appel à un iodure alcalin. En effet les carboxylates en cause semblent limiter l'apparition in situ d'iodure de méthyle (dont on a déjà signalé l'effet néfaste dans le présent procédé) susceptible de se former par réaction d'un iodure alcalin sur l'acétate de méthyle (matière de départ) selon le schéma réactionnel ci-après, la formation d'iodure de méthyle étant plus importante au départ d'iodure de lithium qu'au départ d'une quantité équivalente d'iodure de sodium ou de potassium :

$$A^+I^- + CH_3COOCH_3 \longrightarrow CH_3I + A^+(OCOCH_3)^-$$

Il n'est pas nécessaire que le carboxylate $A'^{n+}(OCOR)_n^-$ dérive du même cation que l'iodure ionique utilisé. Le carboxylate est avantageusement un acétate.

On constatera que certains acétates peuvent être considérés comme le produit d'addition de l'acétate de méthyle et d'une phosphine, d'une amine, d'une arsine ou d'une stibine selon le schéma donné dans le cas particulier de la triphénylphosphine pour simplifier l'exposé :

$$CH_3COOCH_3 + (C_6H_5)_3P \longrightarrow (CH_3)(C_6H_5)_3P^+(OCOCH_3)^-$$

C'est la raison pour laquelle chaque mole de phosphine, d'amine, d'arsine ou de stibine, éventuellement chargée ou alimentée sera assimilée à un équivalent-gramme de cation $A'^{n+}$ dans la condition reliant la quantité totale d'halogène ($X_T$) à celle des cations $A^+$ et $A'^{n+}$ présente dans le milieu réactionnel.

Lorsqu'on utilise un carboxylate du type défini précédemment sa quantité est en général telle que le rapport molaire $A'^{n+}/A^+$ soit compris entre 0,01 et 20. De préférence, ce rapport sera compris entre 0,05 et 10, de manière avantageuse entre 0,1 et 5.

Selon une première variante qui se révèle plus particulièrement avantageuse dans le cas où la source de fer au degré d'oxydation nul est un alliage, le présent procédé est mis en œuvre en présence d'hydrogène. Dans le cadre de cette variante la pression partielle d'hydrogène déterminée à 25 °C sera alors d'au moins 1 bar et de préférence d'au moins 3 bars, et encore mieux, d'au moins 10 bars. En général, la pression partielle d'hydrogène mesurée à 25 °C ne dépassera pas 100 bars ; de manière avantageuse elle sera inférieure à 70 bars et, de préférence, inférieure à 50 bars.

Comme les praticiens le savent, la nature du produit obtenu est fonction de la teneur en eau du milieu réactionnel. Lorsqu'on opère dans un milieu réactionnel qui, compte-tenu des diverses contraintes industrielles, renferme le moins d'eau possible (milieu pratiquement anhydre) on obtient sélectivement l'anhydride acétique. Par contre lorsque le milieu renferme une proportion notable d'eau, on peut obtenir de l'acide acétique en une proportion correspondant à celle de l'anhydride acétique obtenu dans un milieu pratiquement anhydre. Dans la mesure où il s'avère économiquement plus intéressant de produire de l'anhydride acétique, le présent procédé sera conduit dans un milieu pratiquement anhydre.

Comme indiqué en tête du présent mémoire il est nécessaire que la quantité totale de composés halogénés présente dans le milieu réactionnel ($X_T$, exprimée en atome-gramme d'halogène) soit telle que le rapport (atomique) $X_T/(A^+ + n \cdot A'^{n+})$ soit inférieur ou égal à 1.

Cette condition n'exclut pas la possibilité de faire appel à des halogènes ($X_2$), à des acides halogénés (HX), à des halogénures d'alkyle (RX), ou à des halogénures de cobalt ($CoX_2$), mais elle implique que si l'on charge ou alimente ces types de composés on devra nécessairement charger ou alimenter soit un carboxylate $[A'^{n+}(OCOR)_n^-]$ défini précédemment, soit une phosphine, une amine, une arsine ou une stibine, en quantité au moins équivalente à la quantité d'atomes-grammes d'halogène (X) introduite, le cas échéant, au moyen des composés halogénés mentionnés ci-avant. On admet que dans les conditions réactionnelles, les composés halogénés $X_2$, HX, et $CoX_2$ vont réagir sur l'acétate de méthyle pour donner naissance à un halogénure de méthyle, qui réagira à son tour (ainsi que les halogénures d'alkyle RX) sur la phosphine, l'amine, l'arsine ou la stibine pour former l'halogénure d'onium quaternaire correspondant.

C'est la raison pour laquelle, dans l'hypothèse où on fait appel aux composés halogénés de types $X_2$, HX, RX, et $CoX_2$, chaque mole de phosphine, d'amine, d'arsine ou de stibine introduite pour « neutraliser » en quelque sorte ces sources d'halogène sera considérée comme un équivalent-gramme de cation $A^+$ dans la condition reliant la quantité totale d'halogène ($X_T$) à celle des cations $A^+$ et $A'^{n+}$ présente dans le milieu réactionnel.

L'halogénure de méthyle $CH_3X$, au même titre que les halogénures d'alkyle (RX) peut par ailleurs réagir sur les carboxylates $[A'^{n+}(OCOR)_n^-]$ pour donner naissance aux halogénures ioniques correspondants $(A'^{n+}X_n^-)$.

Les phosphines, amines, arsines et stibines auxquelles on recourra, le cas échéant, peuvent être représentées par la formule (IV) ci-après :

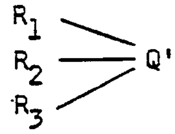 (IV)

dans laquelle Q' représente un atome de phosphore, d'azote, d'arsenic ou d'antimoine, et $R_1$ à $R_3$ ont la même signification que précédemment.

On recourt avantageusement à une phosphine, et plus particulièrement à la triphénylphosphine.

Selon une seconde variante de la présente invention, qui se révèle particulièrement avantageuse dans le cas où la source de fer au degré d'oxydation nul est un alliage, et dans le cas où l'on choisit de mettre en œuvre le présent procédé à une température supérieure ou égale à 160 °C, voire supérieure à 180 °C, la pression totale en température étant supérieure ou égale à 100 bars, le milieu réactionnel initial renferme outre de l'acétate de méthyle et les divers constituants du système catalytique définis dans le présent mémoire, un solvant choisi parmi les acides carboxyliques aliphatiques ayant au maximum 8 atomes de carbone. Parmi ces acides la Demanderesse préconise l'emploi de l'acide acétique. Pour une bonne mise en œuvre du procédé selon l'invention, l'acide carboxylique représente de 1 à 75 % en volume du milieu réactionnel, et encore mieux de 5 à 30 % (en volume) dudit milieu.

Le milieu réactionnel initial peut également renfermer de 10 à 50 % en volume d'un solvant additionnel choisi dans le groupe constitué par la tétraméthylènesulfone, la tétraméthylurée, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone et l'anhydride acétique.

La pression partielle du monoxyde de carbone mesurée à 25 °C est en général supérieure à 10 bars et, de préférence supérieure à 30 bars.

La température de réaction qui peut varier dans de larges limites est en général comprise entre 80 et 240 °C, et de manière avantageuse à une température supérieure à 100 °C, gamme dans laquelle le système catalytique développe une efficacité optimale.

Un avantage du présent procédé réside dans la possibilité de carbonyler efficacement l'acétate de méthyle même avec une faible concentration de cobalt, de fer au degré d'oxydation nul et d'iodure ionique, sous une pression totale en température de l'ordre de 20 à 300 bars, qui est donc bien inférieure à celle habituellement préconisée pour des systèmes catalytiques à base de cobalt.

Un autre avantage du présent procédé réside dans la disparition des diverses contraintes associées à l'utilisation des systèmes catalytiques récemment proposés pour réaliser cette carbonylation.

Comme les spécialistes concernés le savent parfaitement bien, l'acétate de méthyle, matière de départ dans le présent procédé, peut être formé in situ à partir de diméthyléther, aussi est-il tout à fait possible dans le cadre de la présente invention, de charger ou d'alimenter du diméthyléther ou un mélange de diméthyléther et d'acétate de méthyle.

En fin d'opération, les produits obtenus peuvent être aisément séparés, par distillation fractionnée du mélange résultant, par exemple.

Les exemples ci-après illustrent l'invention sans toutefois en limiter le domaine ou l'esprit.

Dans les exemples 1 à 33 ainsi que dans les essais témoins (a) à (f) le mode opératoire utilisé est le suivant :

Dans un autoclave de 125 ml de capacité (en Hastelloy B2 pour les exemples 24 à 31 et en tantale pour tous les autres exemples et les essais témoins), on charge de l'acétate de méthyle, un ou plusieurs solvants, le cas échéant, et les divers constituants du système catalytique. Après fermeture de l'autoclave on admet une pression de monoxyde de carbone et une pression d'hydrogène, respectivement désignées par P(CO) et P($H_2$) (valeurs mesurées à 25 °C).

L'agitation, par un système de va-et-vient, est mise en route et l'autoclave est alors porté à la température choisie et désignée par T, en 25 minutes environ. La pression totale en température, désignée par P(T), est maintenue sensiblement à la valeur indiquée par des recharges successives de monoxyde de carbone renfermant au maximum 1 % (en volume) d'hydrogène. Après une durée de réaction désignée par t, on refroidit l'autoclave et on le dégaze. Le mélange réactionnel est alors analysé par chromatographie et potentiométrie.

Dans les exemples les conventions suivantes sont utilisées :

— AcOMe : désigne l'acétate de méthyle
— AcOH : désigne l'acide acétique
— $Ac_2O$ : désigne l'anhydride acétique
— TMS : désigne la tétraméthylènesulfone
— NMP : désigne la N-méthylpyrrolidone
— TMU : désigne la tétraméthylurée
— Pr : désigne la productivité exprimée en gramme d'anhydride acétique par heure et par litre
— RR désigne le nombre de moles d'anhydride acétique formées pour 100 moles d'acétate de méthyle chargées

— mAt-g : signifie milliatome-gramme
— mMol : signifie millimole

Toutes les pressions [P(CO), P($H_2$) et P(T)] sont exprimées en bars.

Exemples 1 à 12 — Essais témoins (a) à (c) :

Dans le tableau (I) ci-après on a rassemblé les conditions particulières et les résultats obtenus lors d'une série d'essais dont les conditions communes sont les suivantes :

On opère en présence de 1 mAt-g de cobalt engagé sous forme de dicobaltoctacarbonyle, soit une concentration de 20 mAt-g/l.

On utilise 15 mMol d'iodure de méthyltriphénylphosphonium (iodure ionique ; $I^-$/Co = 15) et du fer en poudre, sauf mentions contraires.

T = 210 °C ; P(T) = 250 bars ; $X_T/(A^+ + n \cdot A'^{n+}) = 1$ sauf mentions contraires.

Tableau (I)

| Ex. n° | AcOMe (ml) | AcOH (ml) | Additif nature | Additif ml | Fe mAt-g | P(CO) | P($H_2$) | t mn | $Ac_2O$ g | $Ac_2O$ Pr |
|---|---|---|---|---|---|---|---|---|---|---|
| a | 25 | 5 | NMP | 15 | 0 | 130 | 10 | 35 | 6,49 | 220 |
| 1 | " | " | " | " | 89 | " | " | 20 | 13,70 | 820 |
| b | " | " | " | " | " | " | " | 30 | 0,22 | 10 |
| 2 | " | " | " | " | " | 139 | ~ 1,4 | 20 | 13,16 | 790 |
| 3 | " | 0 | " | " | " | 130 | 10 | " | 13,31 | 800 |
| 4 | 40 | 5 | néant | 0 | 54 | " | " | 30 | 10,15 | 405 |
| c | 40 | " | " | 0 | " | 130 | " | " | 7,18 | 290 |
| 5 | 25 | " | TMS | 15 | " | 139 | ~ 1,4 | " | 11,95 | 480 |
| 6 | " | " | " | " | " | 140 | 0 | " | 12,24 | 490 |
| 7 | 30 | 0 | " | " | " | 140 | 0 | " | 12,34 | 495 |
| 8 | 25 | 5 | NMP | 15 | 18 | 130 | 10 | " | 6,16 | 250 |
| 9 | " | " | " | " | 30 | 84 | 6 | 60 | 15,3 | 300 |
| 10 | " | " | " | " | 89 | 36 | ~ 0,5 | 30 | 3,57 | 140 |
| 11 | 35 | 5 | $H_2O$ | 5 | 54 | 130 | 10 | " | 15,68 | (NB) |
| 12 | 25 | 5 | NMP | 15 | 53 | 139 | ~ 1,4 | " | 9,82 | 390 |

b) : cet essai est réalisé en l'absence de cobalt.

c) : dans cet essai on a chargé en outre 3,8 mMol d'iodure de méthyle $[X_T/(A^- + n \cdot A'^{n-}) = 1{,}25]$.

Dans l'exemple 8 on a utilisé une poudre Fer-Chrome (18 mAt-g de Fer et 77 mAt-g de Chrome).

Dans l'exemple 9 on a utilisé une poudre Fer-Titane (30 mAt-g de Fer et 30 mAt-g de Titane) ; P(T) = 150 bars.

L'exemple 10 est réalisé sous 80 bars de pression totale et avec de l'iodure de potassium au lieu de l'iodure de méthyltriphénylphophonium.

(NB) : on a indiqué dans la colonne précédente le nombre de grammes d'acide acétique dosé.

Dans l'exemple 12, on a chargé en outre 20 mMol d'acétate de sodium

$$[X_T/(A^+ + n \cdot A'^{n+} = 0{,}43]$$

Exemples 13 à 21 — Essais-témoins (d) et (e)

Dans le tableau (II) ci-après, on a rassemblé les conditions particulières et les résultats obtenus lors d'une série d'essais dont les conditions communes sont les suivantes :

On utilise du dicobalt octacarbonyle comme source de cobalt, et sauf mention contraire, l'iodure de méthyltriphénylphosphonium (iodure ionique) et comme source de fer au degré d'oxydation nul un tampon cylindrique en fil d'acier Z8-CNDT 17-12 (norme Afnor) renfermant 69 % de fer, 17 % de chrome, 11,5 % de nickel, 2 % de mobyldène et 0,32 % de titane (les pourcentages sont en poids), ayant une masse spécifique de 525 g/dm³ et une surface d'échange de 215 dm²/dm³).

T = 210 °C sauf dans l'exemple 21 qui est réalisé à 180 °C.

Tableau (II)

| EX n° | AcOMe (ml) | AcOH (ml) | Additif nature | Additif ml | Co mAt-g/l | $I^-/Co$ | Tampon (g) | P(CO) | $P(H_2)$ | P(T) | t mn | $Ac_2O$ g | $Ac_2O$ Pr |
|---|---|---|---|---|---|---|---|---|---|---|---|---|---|
| d | 35 | 10 | néant | 0 | 20 | 15 | 0 | 130 | 10 | 250 | 36 | 1,58 | 55 |
| 13 | " | " | " | " | " | " | 32,5 | " | " | " | 20 | 5,85 | 350 |
| e | 25 | 5 | NMP | 15 | " | " | 0 | " | " | " | 35 | 6,52 | 220 |
| 14 | " | " | " | " | " | " | 16 | 139 | = 1,4 | " | 20 | 9,12 | 545 |
| 15 | " | " | " | " | " | " | =30 à 35 | " | " | " | " | 10,02 | 600 |
| 16 | " | " | " | " | 40 | 7,5 | " | " | " | " | " | 10,24 | 615 |
| 17 | " | " | " | " | 10 | 30 | " | " | " | " | " | 10,49 | 630 |
| 18 | " | " | " | " | 20 | 15 | " | 77 | = 0,8 | 150 | 30 | 8,19 | 325 |
| 19 | " | " | " | " | " | " | " | 36 | = 0,5 | 80 | " | 4,61 | 185 |
| 20 | " | " | " | " | " | " | " | 130 | 10 | 250 | 20 | 6,20 | 370 |
| 21 | 30 | 10 | $H_2O$ | 5 | " | " | " | 139 | = 1,4 | " | " | 21,1 | (xx) |

Dans l'exemple 20, l'iodure ionique utilisé est KI.

(XX) : On a indiqué dans la colonne précédente le nombre d'acide acétique dosé

Exemples 22 à 29

Dans le tableau (III) ci-après, on a rassemblé les conditions particulières et les résultats obtenus lors d'une série d'essais dont les conditions communes sont les suivantes :

On opère en présence de 1 mAt-g de cobalt (20 mAt-g/l) engagé sous forme de dicobaltoctacarbonyle, de 15 mMol d'iodure de méthyltriphénylphosphonium ($I^-/Co = 15$) et, comme source de fer au degré d'oxydation nul, d'un tampon cylindrique d'acier Z8 CNDT 17-12 décrit précédemment.

T = 212 °C ; P(T) = 150 bars ; P(CO) = 77 ; P($H_2$) = 1 ; t = 2 heures.

Tableau (III)

| Ex n° | AcOMe ml | AcOH ml | Additif nature | Additif ml | Tampon (g) | $Ac_2O$ (g) | $Ac_2O$ RR |
|---|---|---|---|---|---|---|---|
| 22 | 40 | 5 | - | 0 | 35,3 | 5,51 | 10,9 |
| 23 | 25 | " | TMS | 15 | 35,2 | 7,65 | 24 |
| 24 | " | " | NMP | " | 35 | 11,73 | 36,7 |
| 25 | " | " | TMU | " | 34,9 | 4,90 | 15,1 |
| 26 | 30 | 0 | NMP | " | 31,3 | 4,33 | 11,3 |
| 27 | " | " | TMS | " | 36,5 | 4,89 | 12,8 |
| 28 | 25 | 5 | NMP | " | 37,9 | 7,85 | 24,6 |
| 29 | 30 | 0 | NMP | " | 34,6 | 3,37 | 8,8 |

Exemples 30 à 32

On a réalisé une série d'essais selon le mode opératoire décrit précédemment sur une charge renfermant :

— 40 ml d'AcOMe
— 5 ml d'AcOH
— du dicobaltoctacarbonyle
— du fer en poudre
— de l'iodure de méthyltriphénylphosphonium (exemples 30 et 32)

ou de l'iodure de tétraéthylammonium (exemple 31).

Les conditions communes sont les suivantes :

P(CO) = 139
P($H_2$) = 1,4
T = 212 °C
P(T) = 250 bars
t = 30 minutes

Les conditions particulières ainsi que les résultats obtenus sont donnés dans le tableau (IV) ci-après :

Tableau (IV)

| Ex n° | Co mAt-g | Fe m At-g | Fe/Co | $A^+I^-$ mMol | $I^-$/Co | $Ac_2O$ g | $Ac_2O$ g/H x 1 |
|---|---|---|---|---|---|---|---|
| 30 | 2 | 25 | 12,5 | 10 | 5 | 6,14 | 245 |
| 31 | 1 | 53 | 53 | 20 | 20 | 7,12 | 285 |
| 32 | 0,5 | 100 | 200 | 25 | 50 | 15,79 | 630 |

Exemple 33 — Essai témoin (f)

Dans un autoclave en tantale et selon le mode opératoire décrit précédemment, on réalise deux essais sur une charge renfermant :

— 30 ml d'AcOMe
— 20 ml de NPM
— 1,5 mAt-g de cobalt engagé sous forme de dicobaltoctacarbonyle
— 30 mMol d'iodure de lithium.

Dans l'exemple 33, la charge renferme en outre 54 mAt-g de fer en poudre.

Les conditions communes sont les suivantes :

— P(CO) = 32 bars
— $P(H_2) = 0$
— T = 152 °C
— P(T) = 50

Pour chaque essai, on enregistre l'absorption du monoxyde de carbone. Dans l'essai témoin (f) on constate qu'il n'y a plus d'absorption au bout de 45 minutes de réaction en température, l'essai est cependant poursuivi. Dans l'exemple 33 on ne constate pas d'arrêt de l'absorption pendant toute la durée de l'essai en température.

Les conditions particulières ainsi que les résultats obtenus sont rassemblés dans le tableau V ci-après.

Tableau V

| Référence | Exemple 33 | Témoin (f) |
|---|---|---|
| Fer (mAt-g) | 54 | 0 |
| T (mn) | 364 | 256 |
| $Ac_2O$ (g) | 20,71 | 7,95 |
| Durée absorption (mn) | > 364 | 45 |

Exemples 34 à 44

Dans ces exemples, le mode opératoire est le suivant :

Dans un autoclave de 300 $cm^3$ en acier inoxydable muni d'une agitation centrale à entraînement magnétique, chauffé et régulé électriquement on introduit :

— 83 ml d'acétate de méthyle,
— 17 ml d'acide acétique,
— 50 ml de N-méthylpyrrolidone,
— 50 mMol d'iodure de méthyltriphénylphosphonium,
— 35 g d'un tampon en acier inoxydable Z8 CNDT 17-12 décrit précédemment,
— du dicobalt octacarbonyle.

On chauffe, sous balayage de monoxyde de carbone et, le cas échéant d'hydrogène jusqu'à la température T. La pression dans l'autoclave est maintenue à 235 bars et le débit d'alimentation du mélange gazeux est de 40 l/h (CNTP). Le pourcentage molaire d'hydrogène dans ce débit d'alimentation est maintenu cosntant et est indiqué dans le tableau (VI) ci-après.

On effectue périodiquement des prélèvements de la masse réactionnelle que l'on analyse.

A partir de ces analyses on détermine graphiquement pour chaque exemple la valeur de k (constante de vitesse) en portant en ordonnées les valeurs de Log (RR) et en abscisse le temps de réaction en température (t). La pente de la droite expérimentale Log (RR) = f(t) est égale à la constante de vitesse k exprimée en heure $^{-1}$, ($h^{-1}$).

Dans le tableau (VI) ci-après on a rassemblé les conditions particulières ainsi que les valeurs respectives de k pour chaque exemple.

Tableau (VI)

| Exemple n° | % $H_2$ | T °C | Co m At-g/l | $I^-$/Co | k($h^{-1}$) |
|---|---|---|---|---|---|
| 34 | 0 | 200 | 11,1 | 30 | 0,19 |
| 35 | 0,5 | " | " | " | 0,30 |
| 36 | 1,2 | " | " | " | 0,49 |
| 37 | 2,6 | " | " | " | 0,60 |
| 38 | 4,3 | " | " | " | 0,63 |
| 39 | 10,3 | " | " | " | 0,80 |
| 40 | 4,3 | 180 | " | " | 0,35 |
| 41 | 10,3 | " | " | " | " |
| 42 | " | 200 | 33,5 | 10 | 0,78 |
| 43 | " | " | 5,5 | 60 | 0,65 |
| 44 | " | " | 2,8 | 120 | 0,53 |

**Revendications**

1. Procédé de préparation de l'anhydride acétique par réaction de l'acétate de méthyle et du monoxyde de carbone en phase liquide, en milieu anhydre en présence :

a) d'une source de cobalt et d'un promoteur halogéné, caractérisé en ce que la réaction est conduite à une température comprise entre 80 et 240 °C sous une pression totale comprise entre 20 et 300 bars en présence également :

b) d'une source de fer au degré d'oxydation nul,

c) d'un iodure ionique de formule :

$$A^+I^-$$

dans laquelle $A^+$ est un cation choisi dans le groupe constitué par les cations onium quaternaire dérivant des éléments du groupe de l'azote et les cations des métaux alcalins, et

d) le cas échéant, d'un carboxylate de formule :

$$A'^{n+}(OCOR)_n^-$$

dans laquelle n est égal à 1 ou 2 et $A'^{n+}$ a la signification donnée ci-avant pour $A^+$, $A'^{n+}$ et $A^+$ pouvant par ailleurs être identiques ou différents, $A'^{n+}$ pouvant, en outre, représenter un cation alcalino-terreux, et R est un radical alkyle, aralkyle ou aryle ayant au maximum 8 atomes de carbone, la quantité totale de composés halogénés présente dans le milieu réactionnel (exprimée en atome-gramme d'halogène et désignée par $X_T$) étant telle que le rapport atomique $X_T/(A^+ + n \cdot A'^{n+})$ soit inférieur ou égal à 1.

2. Procédé selon la revendication 1, caractérisé en ce que la source de fer au degré d'oxydation nul est chosie parmi le fer métallique, $Fe(CO)_5$ et les alliages dans lesquels le fer représente au moins 20 % en poids.

3. Procédé selon la revendication 2, caractérisé en ce que la source de fer au degré d'oxydation nul est le fer métallique.

4. Procédé selon la revendication 1 ou 2, caractérisé en ce que la soruce de fer au degré d'oxydation nul est un alliage de fer dans lequel le fer représente au moins 20 % (en poids).

5. Procédé selon l'une quelconque des revendications précédentes, caractérisé en ce que l'on opère également en présence d'hydrogène.

6. Procédé selon la revendication 1, caractérisé en ce que l'iodure ionique est un iodure de phosphonium quaternaire ou d'ammonium quaternaire.

7. Procédé selon l'une quelconque des revendications précédentes, caractérisé en ce que la concentration en cobalt est comprise entre 0,1 et 500 mAt-g/l.

8. Procédé selon l'une quelconque des revendications précédentes, caractérisé en ce que le milieu réactionnel renferme un solvant choisi parmi les acides carboxyliques aliphatiques ayant au maximum 8 atomes de carbone.

9. Procédé selon la revendication 8, caractérisé en ce que le solvant est l'acide acétique.

10. Procédé selon l'une quelconque des revendications précédentes, caractérisé en ce que le rapport molaire $I^-/Co$ est supérieur ou égal à 5 et, de préférence supérieur ou égal à 10.

11. Procédé selon l'une quelconque des revendications précédentes, caractérisé en ce que le rapport molaire $I^-/Co$ est compris entre 15 et 100.

12. Procédé selon l'une quelconque des revendications précédentes, caractérisé en ce que le rapport atomique Fe/Co est supérieur ou égal à 1 et, de préférence supérieur ou égal à 5.

13. Procédé selon l'une quelconque des revendications précédentes, caractérisé en ce que le rapport atomique Fe/Co est supérieur ou égal à 10.

14. Procédé selon la revendication 12 ou 13, caractérisé en ce que le rapport atomique Fe/Co est inférieur ou égal à 1 000.

15. Procédé selon l'une quelconque des revendications précédentes, caractérisé en ce que la concentration en cobalt est comprise entre 0,5 et 100 mAt-g/l.

**Claims**

1. Process for preparing acetic anhydride by reacting methyl acetate and carbon monoxide in the liquid phase in anhydrous medium in the presence of :

a) a source of cobalt and a halogen-containing promoter, characterised in that the reaction is performed at a temperature between 80 and 240 °C at a total pressure between 20 and 300 bars in the presence also of :

b) a source of iron at zero oxidation number,

c) an ionic iodide of formula :

$$A^+ I^-$$

in which $A^+$ is a cation chosen from the group consisting of the quaternary onium cations derived from elements of the nitrogen group and the alkali metal cations, and

d) where appropriate, a carboxylate of formula :

$$A'^{n+}(OCOR)_n^-$$

in which n equals 1 or 2 and $A'^{n+}$ has the significance given above for $A^+$, where $A'^{n+}$ and $A^+$ can, moreover, be identical or different and $A'^{n+}$ can, in addition, denote an alkaline earth cation, and R is an alkyl, aralkyl or aryl radical having a maximum of 8 carbon atoms, the total amount of halogen-containing compounds present in the reaction medium (expressed in gram-atom of halogen and designated by $X_T$) being such that the atomic ratio $X_T(A^+ + n.A'^{n+})$ is less than or equal to 1.

2. Process according to Claim 1, characterised in that the source of iron at zero oxidation number is chosen from metallic iron, $Fe(CO)_5$ and alloys in which iron represents at least 20 % by weight.

3. Process according to Claim 2, characterised in that the source of iron at zero oxidation number is metallic iron.

4. Process according to Claim 1 or 2, characterised in that the source of iron at zero oxidation number is an alloy of iron in which iron represents at least 20 % (by weight).

5. Process according to any one of the preceding claims, characterised in that the procedure is also performed in the presence of hydrogen.

6. Process according to Claim 1, characterised in that the ionic iodide is a quaternary phosphonium or quaternary ammonium iodide.

7. Process according to any one of the preceding claims, characterised in that the cobalt concentration is between 0.1 and 500 mg-At/l.

8. Process according to any one of the preceding claims, characterised in that the reaction medium contains a solvent chosen from aliphatic carboxylic acids having a maximum of 8 carbon atoms.

9. Process according to Claim 8, characterised in that the solvent is acetic acid.

10. Process according to any one fo the preceding claims, characterised in that the mole ratio $I^-/Co$ is greater than or equal to 5, and preferably greater than or equal to 10.

11. Process according to any one of the preceding claims, characterised in that the mole ratio $I^-/Co$ is between 15 and 100.

12. Process according to any one of the preceding claims, characterised in that the atomic ratio Fe/Co is greater than or equal to 1, and preferably greater than or equal to 5.

13. Process according to any one fo the preceding claims, characterised in that the atomic ratio Fe/Co is greater than or equal to 10.

14. Process according to Claim 12 or 13, characterised in that the atomic ratio Fe/Co is less than or equal to 1.000.

15. Process according to any one of the preceding claims, characterised in that the cobalt concentration is between 0.5 and 100 mg-At/l.

**Patentansprüche**

1. Verfahren zur Herstellung von Essigsäureanhydrid durch Umsetzen von Methylacetat und Kohlenmonoxid in flüssiger Phase in wasserfreiem Medium in Gegenwart von :

a) einer Cobaltquelle und eines Halogenpromotors, dadurch gekennzeichnet, daß die Umsetzung bei einer Temperatur von 80 bis 240 °C unter einem Gesamtdruck von 20 bis 300 bar in Gegenwart von zusätzlich :

b) einer Quelle für Eisen mit Oxidationsgrad 0,

c) eines ionischen Iodids der Formel

$$A^+I^-,$$

in der $A^+$ ein Kation ausgewählt aus der Gruppe bestehend aus den quaternären Oniumkationen der Elemente der Stickstoffgruppe und den Alkalikationen ist und

d) gegebenenfalls eines Carboxylats der Formel

$$A'^{n+}(OCOR)_n^-,$$

in der n 1 oder 2 ist und $A'^{n+}$ die oben für $A^+$ angegebene Bedeutung hat, $A'^{n+}$ und $A^+$ außerdem identisch oder verschieden sein können und $A'^{n+}$ außerdem ein Erdalkalikation bedeuten kann und R eine Alkyl-, Aralkyl- oder Arylgruppe mit maximal 8 Kohlenstoffatomen ist, durchgeführt wird, wobei die gesamtmenge der im Reaktionsmedium vorhandenen halogenierten Verbindungen (angegeben in gAtom Halogen und bezeichnet mit $X_T$) so ist, daß das Atomverhältnis $X_T/(A^+ + n \cdot A'^{n+}) \leq 1$ ist.

2. Verfahren nach Anspruch 1, dadurch gekennzeichnet, daß die Quelle für Eisen mit Oxidationsgrad

0 unter metallischem Eisen, $Fe(CO)_5$ und den Legierungen, in denen das Eisen mindestens 20 Gew.-% ausmacht, ausgewählt wird.

3. Verfahren nach Anspruch 2, dadurch gekennzeichnet, daß die Quelle für Eisen mit Oxidationsgrad 0 metallisches Eisen ist.

4. Verfahren nach Anspruch 1 oder 2, dadurch gekennzeichnet, daß die Quelle für Eisen mit Oxidationsgrad 0 eine Eisenlegierung ist, in der das Eisen mindestens 20 Gew.-% ausmacht.

5. Verfahren nach einem der vorangehenden Ansprüche, dadurch gekennzeichnet, daß man ebenfalls in Gegenwart von Wasserstoff arbeitet.

6. Verfahren nach Anspruch 1, dadurch gekennzeichnet, daß das ionische Iodid ein quaternäres Phosphoniumiodid oder ein quaternäres Ammoniumiodid ist.

7. Verfahren nach einem der vorangehenden Ansprüche, dadurch gekennzeichnet, daß die Cobaltkonzentration 0,1 bis 500 mgAtom/l ausmacht.

8. Verfahren nach einem der vorangehenden Ansprüche, dadurch gekennzeichnet, daß das Reaktionsmedium ein Lösungsmittel ausgewählt aus den aliphatischen Carbonsäuren mit maximal 8 Kohlenstoffatomen umfaßt.

9. Verfahren nach Anspruch 8, dadurch gekennzeichnet, daß das Lösungsmittel Essigsäure ist.

10. Verfahren nach einem der vorangehenden Ansprüche, dadurch gekennzeichnet, daß das Molverhältnis $I^-/Co \geq 5$, vorzugsweise $\geq 10$ ist.

11. Verfahren nach einem der vorangehenden Ansprüche, dadurch gekennzeichnet, daß das Molverhältnis $I^-/Co$ 15 bis 100 beträgt.

12. Verfahren nach einem der vorangehenden Ansprüche, dadurch gekennzeichnet, daß das Atomverhältnis $Fe/Co \geq 1$, vorzugsweise größer $\geq 5$. ist.

13. Verfahren nach einem der vorangehenden Ansprüche, dadurch gekennzeichnet, daß das Atomverhältnis $Fe/Co \geq 10$ ist.

14. Verfahren nach Anspruch 12 oder 13, dadurch gekennzeichnet, daß das Atomverhältnis $Fe/Co \leq$ 1 000 ist.

15. Verfahren nach einem der vorangehenden Ansprüche, dadurch gekennzeichnet, daß die Cobaltkonzentration 0,5 bis 100 mgAtom.l beträgt.